(19) Europäisches Patentamt
European Patent Office
Office européen des brevets

(11) Numéro de publication: **0 279 954 B1**

(12) # FASCICULE DE BREVET EUROPEEN

(45) Date de publication de fascicule du brevet: **17.03.93**

(51) Int. Cl.[5]: **C07C 233/16**, C07D 295/18, C07C 51/353, A61K 31/16, A61K 31/395

(21) Numéro de dépôt: **87119357.9**

(22) Date de dépôt: **30.12.87**

Demande divisionnaire 91101995.8 déposée le 30/12/87.

(54) **Amides de l'acide eicosatétraynoique, leur application en pharmacie et cosmétique, leur préparation et procédé de préparation de l'acide eicosatétraynoique.**

(30) Priorité: **31.12.86 FR 8618419**

(43) Date de publication de la demande:
**31.08.88 Bulletin 88/35**

(45) Mention de la délivrance du brevet:
**17.03.93 Bulletin 93/11**

(84) Etats contractants désignés:
**AT BE CH DE ES FR GB GR IT LI LU NL SE**

(56) Documents cités:
**EP-A- 0 104 468**
**EP-A- 0 161 422**
**EP-A- 0 209 770**
**US-A- 4 190 669**

**CHEMICAL ABSTRACTS, vol. 97, no. 5, 2 août 1982, page 550, abrégé no. 38706a, Columbus, Ohio, US; L. A. YAKUSHEVA et al.: "Synthesis of cis, cis, cis-8,11,14-eicosatrienic and (3H)dihomo-gamma-linoleic acids and their conversion to prostaglandins E1,F1alfa,(3H)-E1alfa-ana(3H)-F1alfa"**

(73) Titulaire: **CENTRE INTERNATIONAL DE RECHERCHES DERMATOLOGIOUES GALDERMA - CIRD GALDERMA**
**Sophia Antipolis**
**F-06560 Valbonne(FR)**

(72) Inventeur: **Shroot, Braham**
**Villa 35 Hameaux de Val Bosquet**
**F-06600 Antibes(FR)**
Inventeur: **Hensby, Christopher**
**89, Chemin D'Andon Villa Madru**
**F-06410 Biot(FR)**
Inventeur: **Maignan, Jean**
**8, Rue Halévy**
**F-93290 Tremblay les Gonesses(FR)**
Inventeur: **Lang, Gérard**
**44, Avenue Lacour**
**F-95210 Saint-Gratien(FR)**
Inventeur: **Restle, Serge**
**140 Rue Anatole France**
**F-93601 Aulnay-sous-Bois(FR)**
Inventeur: **Colin, Michel**
**10 Allée Cécile**
**F-93190 Livry-Gargan(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.5x/3.0.1)

CHEMICAL ABSTRACTS, vol. 96, no. 19, 10 mai 1982, page 169, abrégé no. 156291p, Columbus, Ohio, US; G. I. MYAGKOVA et al.: "Synthesis of arachidonic, tritiated arachidonic acid and its conversion to prostaglandin E2, F2alfa, tritiated E2 and tritiated F1alfa"

CHEMICAL ABSTRACTS, vol. 106, no. 19, 11 mai 1987, page 634, abrégé no. 155871r, Colombus, Ohio, US; Yu.Yu. BELOSLUDTSEV et al.:"A new synthesis of 5,8,11,14-eicosatetraynoic acid"

(74) Mandataire: **Casalonga, Axel et al**
**BUREAU D.A. CASALONGA - JOSSE Morassistrasse 8**
**W-8000 München 5 (DE)**

## Description

La présente invention a pour objet de nouveaux composés constitués par des amides de l'acide eicosatétraynoïque ainsi que leur application, d'une part, comme agents thérapeutiques dans le traitement ou la prophylaxie des maladies allergiques et dans le traitement des dermatoses et des maladies inflammatoires et, d'autre part, dans des compositions cosmétiques.

Il est connu qu'un certain nombre de substances jouent un rôle important dans le processus inflammatoire de la peau telles que l'acné, les dermatoses, comme par exemple le psoriasis, l'eczéma, etc... Ces substances parmi lesquelles les prostaglandines, les acides hydroxyeicosatétraénoïques, les thromboxanes et les leucotriènes, ont toutes une origine commune qui est l'acide arachidonique. (Voir "VOORHEES-Leukotrienes and Other Lipoxygenase Products in the Pathogenesis and Therapy of Psoriasis and Other Dermatoses" Arch Dermatol, Vol. 119, Juillet 1983, 541-547).

La formation de ces substances résulte essentiellement de la libération de l'acide arachidonique lié (par une liaison ester aux lipides présents dans l'épiderme par exemple les phospholipides puis de son oxydation par la cyclooxygénase et/ou les lipooxygénases.

On a déjà préconisé antérieurement, pour le traitement des maladies de la peau, soit des inhibiteurs de la cyclooxygénase empêchant la formation des prostaglandines tels que l'indométhacine, la vitamine E, etc.; ou alors des substances susceptibles d'inhiber les lipoxygénases tels que l'acide eicosatétraynoïque.

On a également préconisé, pour le traitement du psoriasis, l'acide eicosatétraynoïque-5,8,11,14 ainsi que l'acide eicosatriynoïque-5,8,11 et leurs esters d'alkyle inférieurs. (Voir, en particulier, le brevet (US-A-4 190 669).

On a décrit dans la demande EP 104,468 des analogues de l'acide arachidonique à titre d'agents anti-inflammatoires et anti-allergiques. Ces composés sont différents des composés de l'invention car les amides prévus, de façon très générale dans ce document, ne sont pas englobés dans la définition générale des composés de la présente invention.

Compte tenu de la description très large et relativement vague du document de brevet européen EP 104,468, il n'était certainement pas à la portée de l'homme de l'art d'en déduire que les composés de formule différente, non envisagés dans ce document antérieur, peuvent présenter des propriétés d'inhibition du métabolisme enzymatique de l'acide arachidonique par la cyclo-oxygénase et les lipoxygénases.

La demanderesse a découvert que, de façon surprenante, des amides particuliers de l'acide eicosatétraynoïque-5,8,11,14 inhibaient le métabolisme enzymatique de l'acide arachidonique provoqué par la cyclo-oxygénase et les lipoxygénases. Ce résultat est particulièrement inattendu en raison du blocage de la fonction acide sous la forme des amides définis ci-dessous.

Elle a constaté, par ailleurs, que ces composés présentent une biodisponibilité différente de celle des acides correspondants, leur conférant des propriétés thérapeutiques améliorées.

La présente invention a donc pour objet les nouveaux amides de l'acide eicosatétraynoïque-5,8,11,14.

Un autre objet de l'invention est constitué par les compositions pharmaceutiques contenant comme substance active de tels composés.

Un objet de l'invention est également constitué par le procédé de préparation de ces dérivés.

L'invention a par ailleurs pour objet l'utilisation dans le domaine de la cosmétique, de ces composés, notamment dans des compositions antiacnéïques, solaires ou postsolaires ou dans le traitement des dermatites séborrhéiques.

D'autres objets de l'invention apparaîtront à la lecture de la description et des exemples qui suivent.

Les composés conformes à l'invention sont essentiellement caractérisés par le fait qu'ils répondent à la formule :

$$C_5H_{11}-(C\equiv C-CH_2)_4-CH_2CH_2CO\ R \qquad (I)$$

dans laquelle :

R est un groupement amino de structure

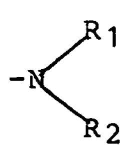

dans laquelle $R_1$ et $R_2$ peuvent être identiques ou différents et correspondent à un atome d'hydrogène, un radical alkyle inférieur en $C_1$-$C_8$, linéaire ou ramifié, substitué au moins par un groupement hydroxyle. Ce radical alkyle inférieur en $C_1$-$C_8$ peut être interrompu par un ou plusieurs hétéroatomes choisis parmi l'oxygène, l'azote ou le soufre;

$R_1$ et $R_2$ ne désignent pas simultanément un atome d'hydrogène;

$R_1$ et $R_2$ peuvent également former avec l'atome d'azote auquel ils sont rattachés, un hétérocycle comportant comme hétéroatome supplémentaire l'azote, l'oxygène ou le soufre, cet hétérocycle étant éventuellement substitué par un groupement alkyl ou hydroxyalkyle, les groupements alkyle ayant de préférence 1 à 8 atomes de carbone; le groupement amino peut également provenir d'un sucre et leurs sels d'acides minéraux ou organiques.

Les composés particulièrement préférés conformes à l'invention sont les composés répondant à la formule (I) dans laquelle, lorsque $R_1$ correspond à un atome d'hydrogène et $R_2$ à une chaîne alkyle substituée par au moins un radical hydroxyle, $R_2$ désigne :

- $CH_2CH_2OH$ (amide de l'éthanolamine)
- $CH_2CHOHCH_2OH$ (amide de la dihydroxy-2,3 propylamine)
- $CH_2CHOHCH_3$ (amide de l'hydroxy-2 propyl amine);

et lorsque la chaîne alkyle $R_2$ est interrompue par un ou plusieurs hétéroatomes, la signification préférée est :

- $CH_2CH_2$-O-$CH_2CH_2OH$ (amide de la diglycol amine).

Lorsque $R_1$ et $R_2$ sont identiques, leur signification préférée est :

- $CH_2CH_2OH$ (amide de la diéthanolamine).

Lorsque $R_1$ et $R_2$ forment ensemble un hétérocycle, les composés préférés de l'invention sont des amides de la morpholine, de la pipérazine ou de l'(hydroxy-2 éthyl)-4 pipérazine.

Lorsque la fontion amino

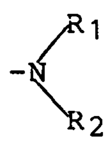

provient d'un sucre, l'amino-sucre préféré est la N-méthylglucamine.

Les composés particulièrement préférés de l'invention sont, notamment :

- le N-(hydroxy-2 éthyloxyéthyl)eicosatétraynamide-5,8,11,14
- le N,N-di(hydroxy-2 éthyl)eicosatétraynamide-5,8,11,14
- le N-(dihydroxy-2,3 propyl)eicosatétraynamide-5,8,11,14
- le N-(hydroxy-2 éthyl)eicosatétraynamide-5,8,11,14
- le N-(hydroxy-2 propyl)eicosatétraynamide-5,8,11,14
- l'(hydroxy-2 éthyl)-4'pipérazino eicosatétraynamide-5,8,11,14 et leurs sels d'acides minéraux ou organiques.

Les composés conformes à l'invention sont préparés de manière générale, à partir de l'acide eicosatétraynoïque-5,8,11,14. Cet acide est connu en lui-même, notamment par le brevet US-A-3.033.884.

Un nouveau procédé de préparation de cet acide eicosatétraynoïque-5,8,11,14 est décrit dans le schéma-A-ci-dessous.

Ce procédé consiste, dans un premier temps, à traiter l'heptyne-1 (1) par un dihalogéno-1,4 butyne (2). Ces deux réactifs (1) et (2) sont connus en euxmêmes. L'acétylure de l'heptyne (1) est formé par échange avec un organomagnésien d'alkyle tel que, de préférence, le bromure d'éthylmagnésium.

Cet acétylure de l'heptyne est mis en réaction avec le dihalogénure (2) que l'on fait réagir en excès. On obtient, de cette façon, l'halogéno-1 undécadiyne-2,5 (3) avec un bon rendement.

L'intérêt principal de ce procédé est donc de passer en une étape d'une chaîne à 7 atomes de carbone à une chaîne à 11 atomes de carbone ayant en position-2 une triple liaison et en position-1 l'atome d'halogène.

Les synthèses de cet halogénure décrites jusqu'à présent consistaient en des réactions d'allongement de chaîne à l'aide de l'alcool propargylique. L'alcool ainsi synthétisé était transformé en halogénure par action d'un trihalogénure de phosphore, ce qui nuisait au rendement du procédé.

Le passage de l'halogénure ayant 11 atomes de carbone de formule (3) à l'hydroxy-1 tétradécatriyne-2,5,8 (5) est réalisé par action du dianion de l'alcool propargylique (4). Cet alcool de formule (5) est à son tour transformé en bromo-1 tétradécatriyne-2,5,8 de formule (6), par action du tribromure de phosphore.

Ces deux dernières étapes sont décrites dans le brevet US-A-3.033.884.

L'acide eicosatétraynoïque-5,8,11,14 est obtenu en faisant réagir le dianion de l'acice hexyne-5 carboxylique suivant un procédé connu, sur le bromo-1 tétradécatriyne-2,5,8 de formule (6). La synthèse de cet acide hexyne-5 carboxylique est décrite en particulier dans la demande de brevet européen EP-A-86.109.150.

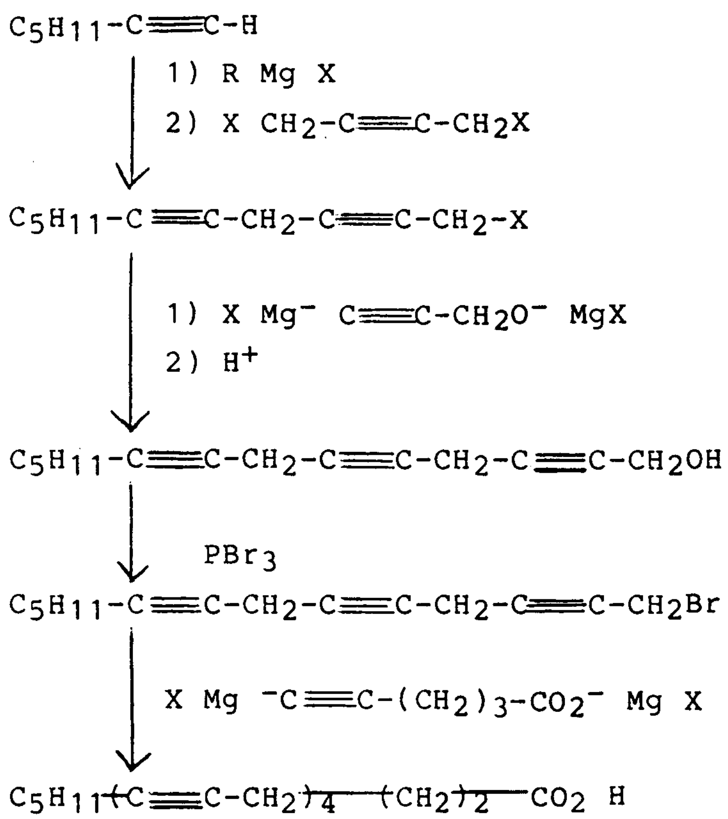


(1) (2) (3) (4) (5) (6) (7) (8)

SCHEMA-A-

Les amides de formule (I) conformes à l'invention sont obtenus en faisant réagir une forme activée de l'acide eicosatétraynoïque-5,8,11,14 sur une amine, dans un solvant organique.

Cette forme activée de l'acide peut être, soit un chlorure d'acide, un anhydride, ou encore l'intermédiaire formé par addition à une solution d'acide de carbonyl diimidazole (C.D.I.).

Cette dernière réaction est conduite de préférence en milieu solvant tel que le diméthylformamide ou encore dans un solvant chloré comme le dichlorométhane ou le dichloro-1,2 éthane. Cette réaction est illustrée par le schéma réactionnel B suivant :

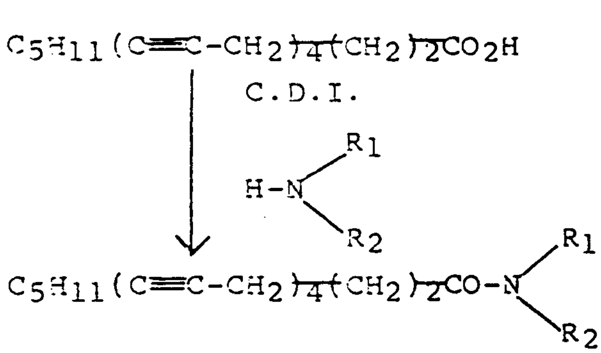


(I)

SCHEMA-B-

Dans la formule (I), $R_1$ et $R_2$ ont les significations indiquées ci-dessus.

Les composés de formule (I), conformes à l'invention, ont une activité particulièrement remarquable vis-à-vis de l'inhibition du métabolisme de l'acide arachidonique et notamment au niveau des lipoxygénases à l'origine de la formation des leucotriènes et des acides hydroxylés qui jouent un rôle important dans le processus inflammatoire.

Les composés conformes à l'invention peuvent être administrés à l'homme ou à l'animal à l'aide de compositions contenant, en plus, un support ou diluant pharmaceutiquement acceptable. Ces compositions peuvent également contenir, si on le souhaite, d'autres substances actives n'ayant pas d'effet antagoniste sur les composés conformes à l'invention.

Les compositions conformes à l'invention peuvent être administrées par voies systémique ou locale.

Pour la voie entérale, les médicaments peuvent se présenter sous forme de comprimés, de gélules, de dragées, de sirops, de suspensions, de solutions, de poudres, de granulés, d'émulsions, de suppositoires, etc. Pour la voie topique, les compositions pharmaceutiques à base de composés selon l'invention se présentent, entre autres, sous forme d'onguents, de teintures, de crèmes, de pommades, de poudres, de timbres, de tampons imbibés, de solutions, de lotions, de gels, de sprays, de shampooings ou encore de suspensions.

Ces compositions utilisées par voie topique peuvent se présenter, soit sous forme anhydre, soit sous forme aqueuse, selon l'indication clinique.

Les compositions pharmaceutiques, conformes à l'invention, peuvent également être administrées par voie parentérale et notamment par voie intraveineuse, intramusculaire, intrapéritonéale, sous-cutanée ou intradermique.

Pour les voies parentérales, et plus particulièrement les injections, on utilise la substance active dans un véhicule stérile tel que l'eau. La substance active est soit mise en suspension ou dissoute dans le véhicule.

Les composés, conformes à l'invention, peuvent également être utilisés en cosmétique, notamment dans les crèmes, les lotions pour la peau telles que dans les produits solaires, post-solaires apaisants, anti-séborrhéiques ou antiacnéiques ou encore de shampooings.

Les compositions médicamenteuses et cosmétiques conformes à l'invention, peuvent contenir des additifs inertes ou pharmacodynamiquement ou cosmétiquement actifs et notamment :

- des agents hydratants comme la thiamorpholinone et ses dérivés ou l'urée; des agents anti-séborrhéiques tels que la S-carboxyméthylcystéine, la S-benzylcystéamine et leurs dérivés, la tioxolone; des antibiotiques comme l'érythromycine et ses esters, la néomycine, la clindamycine et ses dérivés ou les tétracyclines; des agents qui interfèrent avec la kératinisation tels que l'acide salicylique et les acides $\alpha$-hydroxycarboxyliques; des agents favorisant la repousse des cheveux tel que le "Minoxidil" (2,4-diamino 6-pipéridino pyrimidine 3-oxyde) et ses dérivés, le Diazoxyde et le Phénytoïn; d'autres agents anti-inflammatoires stéroïdiens ou non stéroïdiens; des caroténoïdes et notamment le $\beta$-carotène; des agents anti-psoriasiques tels que l'antraline et ses dérivés; des inhibiteurs de phospholipase $A_2$.

Les compositions conformes à l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des antioxydants tels que l'$\alpha$-tocophérol, le butylhydroxyanisole ou le butylhydroxytoluène, l'acide ascorbique, des anesthésiques locaux, des tampons, etc..

Les compositions selon l'invention peuvent également être conditionnées sous des formes retard ou à libération progressive, connues en elles-mêmes. Elles peuvent enfin être introduites dans des phases aqueuses de liposomes ou de niosomes.

La substance active, selon l'invention, est présente dans les compositions pharmaceutiques ou cosmétiques dans des proportions comprises entre 0,01 et 10% en poids par rapport au poids total de la composition et de préférence entre 0,1 et 5% en poids.

Dans l'application thérapeutique, le traitement est déterminé par le médecin et peut varier suivant l'âge, le poids et la réponse du patient ainsi que la gravité des symptômes. Le dosage est généralement compris entre 0,05 et 500 mg/kg/jour et de préférence 0,5 à 100 mg/kg/jour. La durée du traitement est variable suivant la gravité des symptômes et peut s'étaler entre 1 et 12 semaines de façon continue ou discontinue.

Dans l'application cosmétique, les compositions conformes à l'invention sont essentiellement utilisées comme produits solaires, apaisants post-solaires et pour le traitement de dermatites séborrhéiques et/ou acnéiques.

Un autre objet de l'invention est constitué par l'utilisation des composés de formule (I) dans la préparation de compositions pharmaceutiques destinées au traitement ou à la prophylaxie des maladies

allergiques et dans le traitement de l'acné, des dermatoses et des maladies inflammatoires.

Les exemples suivants sont destinés à illustrer l'invention sans pour autant présenter un caractère limitatif.

EXEMPLE DE REFERENCE

Préparation de l'acide eicosatétraynoïque-5,8,11,14

a) Synthèse du chloro-1 undécadiyne-2,5

Dans un ballon équipé d'une arrivée d'argon, d'un réfrigérant et d'une ampoule à brome, introduire 4,17 g (0,17 mole) de magnésium. Recouvrir le magnésium de THF, ajouter quelques gouttes de bromure d'éthyle, amorcer la réaction par chauffage ou par introduction d'un cristal d'iode. Maintenir la réaction au reflux du THF par addition, au goutte à goutte, de 14 $cm^3$ de bromure d'éthyle en solution dans le THF, puis chauffer à 70-80 °C jusqu'à disparition totale du magnésium.

Ajouter 15 g d'heptyne (0,156 mole) en suivant le dégagement d'éthane et en maintenant la température à 70 °C. La réaction est très exothermique. Introduire 1,1 g de cyanure de cuivre et chauffer à 70 °C pendant une heure.

Refroidir le milieu réactionnel à 50 °C et ajouter rapidement 43 $cm^3$ (0,44 mole-2,8 équivalents) de dichlorobutyne. La réaction étant très exothermique, maintenir la température inférieure à 70 °C au cours de l'addition à l'aide d'un bain eau-glace puis chauffer à 70 °C pendant environ 2 heures.

En C.P.V., on constate la disparition totale de l'heptyne.

Verser le milieu réactionnel sur une solution glacée (500 $cm^3$) de chlorure d'ammonium et extraire à l'éther.

Laver la phase organique, sécher sur sulfate de magnésium et concentrer sous pression réduite.

Le produit attendu est purifié par distillation.

L'excès de dichlorobutyne est éliminé par distillation sous trompe à eau et le chloro-1 undécadiyne-2,5 est distillé sous vide de la pompe à palette.

Le produit attendu distille à 95-105 °C sous 0,01 mm Hg. Il est obtenu avec un rendement de 58%.

Le spectre $^1H$ RMN (80 MHz) est en accord avec la structure attendue.

b) Synthèse de l'hydroxy-1 tétradécatriyne-2,5,8

Dans un ballon muni d'une arrivée d'azote et d'une ampoule à brome, introduire 4,8 g de magnésium (0,197 mole). Recouvrir le magnésium de THF, ajouter quelques gouttes de bromure d'éthyle et amorcer la réaction par chauffage ou addition d'un cristal d'iode. Maintenir la réaction au reflux du THF par addition, au goutte à goutte, de 15 $cm^3$ de bromure d'éthyle (0,203 mole) en solution dans du THF, puis chauffer à 70-80 °C jusqu'à disparition totale du magnésium.

Refroidir le milieu réactionnel à 0 °C et ajouter au goutte à goutte, 6,2 $cm^3$ (0,105 mole) d'alcool propargylique distillé et conservé sur tamis moléculaire.

Maintenir la température à 0 °C pendant 30 minutes, puis chauffer à 70 °C pendant une heure pour terminer l'échange.

Refroidir le milieu réactionnel à 0 °C, ajouter 0,4 g de cyanure de cuivre puis chauffer à 40-45 °C pendant environ une heure.

Ajouter une solution de 12 g (0,066 mole) de chloro-1 undécadiyne-2,5 dans du THF et porter pendant environ 20 heures au reflux du THF.

La réaction est suivie en CCM. A la fin du temps de chauffage, il reste des traces de chloro-1 undécadiyne-2,5 de départ.

Verser le milieu réactionnel sur 600 $cm^3$ d'une solution de chlorure d'ammonium. Extraire à l'acétate d'éthyle, laver les phases organiques et sécher sur sulfate de magnésium. Par concentration sous pression réduite, on obtient une huile brune.

Reprendre cette huile dans de l'heptane chaud.

Par cristallisation au congélateur, on obtient 6,8 g (Rendement : 51%) d'une poudre blanche fondant à 26-27 °C.

Le spectre $^1H$ RMN est conforme à la structure attendue.

c) Synthèse du bromo-1 tétradécatriyne-2,5,8

Dans un ballon muni d'une arrivée d'argon, introduire 6 g d'hydroxy-1 tétradécatriyne-2,5,8 préparé précédemment, en solution dans 30 $cm^3$ d'éther éthylique. Ajouter 0,05 $cm^3$ de pyridine et additionner goutte à goutte 1 $cm^3$ de tribromure de phosphore puis porter au reflux pendant 3 heures.

Après avoir vérifié en CCM la fin de la réaction, verser le milieu réactionnel sur de l'eau glacée et extraire le produit attendu à l'éther. Laver les phases organiques avec une solution diluée de carbonate de sodium puis avec de l'eau. Sécher sur sulfate de magnésium et concentrer sous pression réduite.

On récupère 8 g d'une huile brune dont le spectre $^1H$ RMN (80 MHz) correspond à la structure attendue et qui sera utilisée telle quelle pour la suite des réactions.

d) Synthèse de l'acide eicosatétraynoïque-5,8,11,14

Dans un ballon muni d'une arrivée d'argon, d'un réfrigérant et d'une ampoule à brome, introduire 3,8 g (0,156 mole) de magnésium.

Recouvrir le magnésium de THF et réaliser le Grignard en ajoutant 13 $cm^3$ (0,172 mole) de bromure d'éthyle.

Maintenir le chauffage jusqu'à disparition totale du magnésium.

Refroidir le milieu réactionnel à 0 °C et introduire goutte à goutte 7,7 g d'acide hexyne-5 carboxylique en solution dans le THF.

Laisser remonter la température à 25 °C pendant 2 heures. Ajouter 0,6 g de cyanure de cuivre, maintenir l'agitation pendant 2 heures à température ambiante, puis ajouter 8 g (0,03 mole) de bromo-1 tétradécatriyne-2,5,8 obtenu précédemment en solution dans le THF.

Chauffer pendant environ 20 heures au reflux du THF. En CCM, on ne constate plus d'évolution de la réaction.

Verser le milieu réactionnel sur une solution diluée d'acide chlorhydrique dans de la glace. Extraire à l'acétate d'éthyle.

Laver les phases organiques avec un solution d'hydrogéno-carbonate de sodium pour éliminer l'excès d'acide hexyne-5 carboxylique. Laver à nouveau à l'eau puis sécher la phase organique.

Concentrer sous pression réduite puis recristalliser le produit brut ainsi obtenu dans un minimum de méthanol.

On récupère 1,5 g de cristaux légèrement crème dont le point de fusion est de 78-80 °C et qui semblent hygroscopiques.

Les spectres $^1H$ RMN et IR sont conformes avec la structure attendue.

Analyse élémentaire : $C_{20}H_{24}O_2$

| | | C | H | O |
|---|---|---|---|---|
| Valeur théorique | : | 81,04 | 8,16 | 10,72 |
| Valeur trouvée | : | 77,96 | 8,25 | 13,73 |
| Valeur théorique avec 2/3 $H_2O$ | : | 77,95 | 8,21 | 13,85 |

EXEMPLE DE PREPARATION 1

Synthèse du N-(hydroxy-2 éthyloxyéthyl) eicosatétraynamide-5,8,11,14

A une solution de 1,5 g d'acide eicosatétraynoïque-5,8,11,14 (5 mmoles) dans 30 $cm^3$ de DMF anhydre, dégazée à l'argon, ajouter 1,1 g de carbonyldiimidazole et chauffer à 50 °C pendant 1 heure 30 minutes.

Refroidir la solution à 0 °C, puis additionner 1,05 g (0,01 mole) d'hydroxy-2 éthyloxyéthylamine en solution dans 10 $cm^3$ de DMF.

Laisser revenir à température ambiante pendant 2 heures puis, après avoir vérifié en CCM la disparition de l'acide de départ, verser le milieu réactionnel sur une solution diluée de chlorure d'ammonium, extraire à l'acétate d'éthyle. Laver à l'eau les phases organiques. Sécher et concentrer sous pression réduite.

Cristalliser le brut de réaction dans l'heptane.

Par recristallisation dans l'éther diisopropylique, on obtient 1,1 g d'une poudre blanche dont le point de fusion est de 68-69 ° C.

Les spectres $^{13}$C RMN et IR sont conformes à la structure attendue.

Analyse élémentaire : $C_{24}H_{33}NO_3$

| | | C | H | N | O |
|---|---|---|---|---|---|
| Valeur théorique | : | 75,16 | 8,67 | 3,65 | 12,51 |
| Valeur trouvée | : | 74,60 | 8,73 | 3,81 | 13,23 |

EXEMPLE DE PREPARATION 2

Synthèse de l'(hydroxy-2 éthyl)-4'pipérazino eicosatétraynamide-5,8,11,14

A une solution de 1,5 g d'acide eicosatétraynoïque-5,8,11,14 (5 mmoles) dans 30 $cm^3$ de DMF anhydre, dégazée à l'argon, ajouter 1,1 g de carbonyldiimidazole et chauffer à 50 ° C pendant 1 heure 30 minutes.

Refroidir la solution à 0 ° C, puis additionner 1,3 g d'hydroxy-2 éthylpipérazine (0,01 mole) en solution dans 10 $cm^3$ de DMF.

Laisser revenir à température ambiante pendant 2 heures, puis après avoir vérifié la disparition de l'acide de départ en CCM, verser le milieu réactionnel sur une solution diluée de chlorure d'ammonium. Extraire à l'éther diisopropylique, laver les phases organiques abondamment à l'eau pour éliminer l'excès d'hydroxy-2 éthylpipérazine. Sécher et concentrer sous pression réduite.

On récupère 1 g d'une huile brune dont les spectres $^{13}$C RMN et IR sont conformes à la structure attendue.

Analyse élémentaire : $C_{26}H_{28}N_2O_2$

| | | C | H | N | O |
|---|---|---|---|---|---|
| Valeur théorique | : | 77,96 | 7,04 | 6,99 | 7,99 |
| Valeur trouvée | : | 74,65 | 8,54 | 6,94 | 9,99 |
| Valeur théorique avec 3/4 $H_2O$ | : | 75,18 | 7,20 | 6,78 | 10,64 |

EXEMPLE DE PREPARATION 3

Synthèse du N-(hydroxy-2 éthyl)eicosatétraynamide-5,8,11,14

A une solution de 500 mg d'acide eicosatétraynoïque-5,8,11,14 (1,7 mmole) dans 20 $cm^3$ de dichloro-1,2 éthane anhydre, dégazée à l'argon, ajouter 360 mg de carbonyldiimidazole en maintenant la solution à 15 ° C pendant environ 1 heure.

Refroidir la solution à 0 ° C, puis additionner 200 mg d'éthanolamine en solution dans du dichloro-1,2 éthane.

Abandonner pendant une nuit à température ambiante, puis après avoir vérifié en CCM la disparition de l'acide de départ, verser le milieu réactionnel sur une solution diluée d'acide chlorhydrique.

Extraire au dichlorométhane, laver les phases organiques abondamment à l'eau pour éliminer l'excès d'éthanolamine. Sécher et concentrer sous pression réduite.

On récupère 250 mg d'une poudre blanche qui est recristallisée dans l'acétonitrile et dont le point de fusion est de 93-94 ° C.

Les spectres $^1$H RMN (80 MHz) et IR correspondent à la structure attendue.

EXEMPLE DE PREPARATION 4

Synthèse du N-(dihydroxy-2,3 propyl)eicosatétraynamide-5,8,11,14

A une solution de 500 mg d'acide eicosatétraynoïque-5,8,11,14 (1,7 mmole) dans 20 $cm^3$ de dichloro-1,2 éthane anhydre, dégazée à l'argon, ajouter 360 mg de carbonyldiimidazole en maintenant la solution à 15 ° C pendant environ une heure.

Refroidir la solution à 0 ° C, puis additionner 310 mg d'amino-3 propanediol-1,2 en solution dans du dichloro-1,2 éthane. Abandonner une nuit à température ambiante, puis après avoir vérifié en CCM la disparition de l'acide de départ, verser le milieu réactionnel sur une solution diluée d'acide chlorhydrique.

Extraire au dichlorométhane, laver les phases organiques abondamment à l'eau pour éliminer l'excès d'amino-3 propanediol-1,2. Sécher et concentrer sous pression réduite.

On récupère 200 mg d'une poudre blanche qui est purifiée par chromatographie sur gel de silice (éluant : chloroforme/acétate d'éthyle/méthanol) et dont le point de fusion est de 95-96 ° C.

Les spectres $^1H$ RMN (80 MHz) et IR sont conformes à la structure attendue.

Les exemples suivants sont destinés à illustrer des compositions conformes à l'invention.

EXEMPLE 1

On prépare la composition suivante :

| | | |
|---|---|---|
| - N-(hydroxy-2 éthyl) eicosatétraynamide-5,8,11,14 | | 5,0 g |
| - Polyéthylène micronisé | | 10,0 g |
| - Myristate d'isopropyle | qsp | 100,0 g |

Cette composition se présente sous forme d'un onguent hydrophobe destiné à une application topique. On obtient également de bons résultats en remplaçant dans cet onguent le N-(hydroxy-2 éthyl) eicosatétraynamide-5,8,11,14 par le N-(dihydroxy-2,3 propyl) eicosatétraynamide-5,8,11,14 ou encore par le N-(hydroxy-2 éthyloxyéthyl) eicosatétraynamide-5,8,11,14.

EXEMPLE 2

On prépare la composition suivante :

| | | |
|---|---|---|
| - N-(dihydroxy-2,3 propyl) eicosatétraynamide-5,8,11,14 | | 1,0 g |
| - Triglycérides des acides caprique, caprylique et stéarique | | 40,0 g |
| - Triglycérides des acides caprique et caprylique | | 30,0 g |
| - Vaseline | | 20,0 g |
| - Huile de vaseline | qsp | 100,0 g |

Cette composition se présente sous forme d'un onguent hydrophobe destiné à une application topique.

EXEMPLE 3

On prépare la composition suivante :

| | | |
|---|---|---|
| - (Hydroxy-2 éthyl)-4'pipérazino eicosatétraynamide-5,8,11,14 | | 0,5 g |
| - Alcool cétylique | | 6,4 g |
| - Alcool cétylique oxyéthyléné à 20 moles d'oxyde d'éthylène | | 2,1 g |
| - Monostéarate de glycérol | | 2,0 g |
| - Triglycérides des acides caprique et caprylique | | 15,0 g |
| - Propylèneglycol | | 10,0 g |
| - Eau | qsp | 100,0 g |

Cette composition se présente sous forme d'une crème destinée à une application topique.

EXEMPLE 4

On prépare la lotion suivante :

| | | |
|---|---|---|
| - N-(dihydroxy-2,3 propyl) eicosatétraynamide-5,8,11,14 | | 0,1 g |
| - Ethanol | | 50,0 g |
| - Propylèneglycol | qsp | 100,0 g |

Cette lotion est utilisée pour une application topique.

Les compositions des exemples 1 à 4 qui précèdent sont toutes fabriquées et stockées en atmosphère inerte et à l'abri de la lumière.

EXEMPLE 5

On prepare la composition suivante :

| | | |
|---|---|---|
| - N-(dihydroxy-2,3 propyl) eicosatétraynamide-5,8,11,14 | | 0,01 g |
| - Ethanol absolu | | 1,0 ml |
| - Aromatisant qs, conservateur qs, | | |
| - Glycérol | qsp | 5,0 ml |

qui est introduite dans une ampoule en verre brun de 5 ml et destinée à être utilisée par voie orale sous forme d'un soluté buvable.

EXEMPLE 6

On prépare une gélule de 350 mg contenant une poudre ayant la composition suivante :

| | |
|---|---|
| - N-(hydroxy-2 éthyloxyéthyl) eicosatétraynamide-5,8,11,14 | 0,025 g |
| - Cellulose microcristalline | 0,020 g |
| - Amidon de maïs | 0,100 g |
| - Silice colloïdale | 0,020 g |
| - Stéarate de magnésium | 0,185 g |

EXEMPLE 7

On prépare des granulés ayant la composition suivante :

| | |
|---|---|
| - (Hydroxy-2 éthyl)-4'pipérazino-eicosatétraynamide-5,8,11,14 | 0,500 g |
| - Méthyl cellulose | 0,020 g |
| - Eau purifiée | 0,400 g |
| - Saccharose | 1,480 g |

La pâte obtenue par mélange des quatre constituants est granulée par voie humide et séchée.
Les granulés sont présentés en sachets de 2 g, la posologie préconisée est de quatre sachets par jour.

**Revendications**
**Revendications pour les Etats contractants suivants : AT, BE, CH, DE, FR, GB, IT, LI, LU, NL, SE**

**1.** Composé caractérisé par le fait qu'il répond à la formule :

$$C_5H_{11}(C{\equiv}C{-}CH_2)_4CH_2CH_2CO\ R \qquad (I)$$

dans laquelle R est un groupement amino de structure

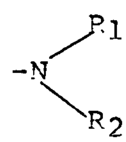

dans laquelle $R_1$ et $R_2$, identiques ou différents, désignent un atome d'hydrogène, un radical alkyle inférieur en $C_1$-$C_8$, linéaire ou ramifié, substitué par au moins un groupement hydroxyle, ce radical alkyle inférieur en $C_1$-$C_8$ pouvant être interrompu par un ou plusieurs hétéroatomes choisis parmi l'oxygène, l'azote ou le soufre, $R_1$ et $R_2$ ne désignant pas simultanément un atome d'hydrogène et $R_1$ et $R_2$ pouvant former avec l'atome d'azote auquel ils sont rattachés, un hétérocycle comportant comme hétéroatome supplémentaire un ou plusieurs atomes d'azote, d'oxygène ou de soufre, l'hétérocycle étant éventuellement substitué par un groupement alkyle ou hydroxyalkyle, le groupement amino pouvant également provenir d'un sucre et leurs sels d'acides minéraux ou organiques.

**2.** Composé selon la revendication 1, caractérisé par le fait qu'il répond à la formule (I), dans laquelle R désigne le groupement :

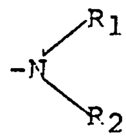

dans laquelle $R_1$ désigne hydrogène et $R_2$ un groupement :

$-CH_2CH_2-OCH_2CH_2OH$,$-CH_2CH_2OH$,$-CH_2-CHOHCH_3$ ou $-CH_2CHOHCH_2OH$ .

**3.** Composé selon la revendication 1, caractérisé par le fait que $R_1$ et $R_2$ forment un cycle dérivé de la morpholine, de la pipérazine et de l'hydroxy-2'éthyl-4 pipérazine.

**4.** Composé selon la revendication 2, caractérisé par le fait qu'il s'agit du N-(hydroxy-2 éthyloxyéthyl) eicosatétraynamide-5,8,11,14.

**5.** Composé selon la revendication 2, caractérisé par le fait qu'il s'agit du N-(hydroxy-2 éthyl) eicosatétraynamide-5,8,11,14.

**6.** Composé selon la revendication 2, caractérisé par le fait qu'il s'agit du N-(dihydroxy-2,3 propyl) eicosatétraynamide-5,8,11,14.

**7.** Composé selon la revendication 3, caractérisé par le fait qu'il s'agit de l'(hydroxy-2 éthyl)-4'pipérazino eicosatétraynamide-5,8,11,14.

**8.** Procédé de préparation des composés selon l'une quelconque des revendications 1 à 7, caractérisé par le fait que l'on transforme l'acide eicosatétraynoïque-5,8,11,14 en chlorure d'acide ou en anhydride d'acide correspondant, sur lequel on fait réagir en présence d'une amine tertiaire une amine de formule :

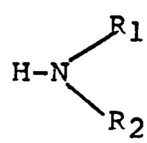

dans laquelle
$R_1$ et $R_2$ ont les significations indiquées dans la revendication 1 ou
que l'on fait réagir sur l'acide eicosatétraynoïque-5,8,11,14, le carbonyldiimidazole en présence d'un solvant et que l'on ajoute ensuite un excès d'amine de formule :

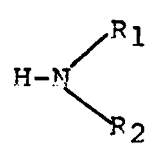

dans laquelle $R_1$ et $R_2$ ont les significations indiquées dans la revendication 1.

**9.** Procédé de préparation selon la revendication 8, caractérisé par le fait que l'on utilise l'acide eicosatétraynoïque-5,8,11,14, préparé selon le procédé où l'on traite l'heptyne-1 répondant à la formule :

$C_5H_{11}$-C≡C-H (1)

avec une base forte pour former l'acétylure correspondant qui est amené à réagir avec le dihalogéno-1,4-butyne-2, de formule :

$XCH_2\text{-}C\equiv C\text{-}CH_2X$ (2)

pour obtenir l'halogéno-1 undécadiyne-2,5 de formule :

$C_5H_{11}\text{-}C\equiv C\text{-}CH_2\text{-}C\equiv C\text{-}CH_2X$ (3)

que l'on amène à réagir avec le dianion de l'alcool propargylique, le produit résultant étant bromé par l'intermédiaire du tribromure de phosphore pour obtenir le bromo-1 tétradécatriyne-2,5,8 de formule :

$C_5H_{11}\text{-}C\equiv C\text{-}CH_2\text{-}C\equiv C\text{-}CH_2\text{-}C\equiv C\text{-}CH_2\text{-}Br$ (6)

qui est mis à réagir avec le dianion de l'acide hexyne-5 carboxylique pour obtenir l'acide eicosatétraynoïque.

**10.** Médicament caractérisé par le fait qu'il contient un composé tel que défini dans l'une quelconque des revendications 1 à 7.

**11.** Composition pharmaceutique destinée à être administrée par voie systémique ou par voie locale, caractérisée par le fait qu'elle contient en présence d'un support ou d'un diluant pharmaceutiquement acceptable, au moins un composé tel que défini dans l'une quelconque des revendications 1 à 7.

**12.** Composition pharmaceutique destinée à être administrée par voie topique, se présentant sous forme de crèmes, de teintures, d'onguents, de pommades, de poudres, de timbres, de tampons imbibés, de solutions, de gels, de lotions, de sprays, de suspensions, caractérisée par le fait qu'elle contient au moins un composé tel que défini dans l'une quelconque des revendications 1 à 7.

**13.** Composition pharmaceutique se présentant sous forme de composition destinée à être administrée de façon parentérale par voie intraveineuse, intrapéritonéale, intramusculaire, sous-cutanée ou intradermique, caractérisée par le fait qu'elle contient un composé tel que défini dans l'une quelconque des revendications 1 à 7, dans un diluant pharmaceutiquement acceptable.

**14.** Composition pharmaceutique destinée à être administrée par voie entérale, se présentant sous forme de comprimés, de gélules, de dragées, de sirops, de suspensions, de solutions, de poudres, de granulés, d'émulsions, caractérisée par le fait qu'elle contient un composé tel que défini dans l'une quelconque des revendications 1 à 7.

**15.** Composition pharmaceutique selon l'une quelconque des revendications 11 à 13, caractérisée par le fait qu'elle contient le composé actif de formule (I) dans les proportions de 0,01 à 10% en poids par rapport au poids total de la composition, et de préférence entre 0,1 et 5% en poids.

**16.** Utilisation d'un composé tel que défini dans l'une quelconque des revendications 1 à 7 pour la préparation d'un médicament destiné au traitement et à la prophylaxie des maladies allergiques et au traitement des dermatoses et des maladies inflammatoires telles que le psoriasis, l'eczéma, l'acné.

**17.** Utilisation selon la revendication 16 d'un composé défini dans l'une quelconque des revendications 1 à 7 pour la préparation d'un médicament destiné au traitement et à la prophylaxie des maladies allergiques et au traitement des dermatoses et des maladies inflammatoires telles que le psoriasis, l'eczéma, l'acné, à des doses de 0,05 à 500 mg/kg/jour et de préférence 0,5 à 100 mg/kg/jour.

**18.** Composition cosmétique caractérisée par le fait qu'elle contient au moins un composé toi que défini dans l'une quelconque des revendications 1 à 7, dans une proportion comprise entre 0,01 et 10% en poids et de préférence entre 0,1 et 5% en poids par rapport au poids total de la composition, en présence d'un support cosmétiquement acceptable.

**19.** Application d'un composé tel que défini dans l'une quelconque des revendications 1 à 7 comme produit cosmétique.

**Revendications pour les Etats contractants suivants : ES, GR**

**1.** Procédé de préparation des composés répondant à la formule générale (I) :

$$C_5H_{11}(C\equiv C-CH_2)_4CH_2CH_2CO\ R \qquad (I)$$

dans laquelle :

R est un groupement amino de structure

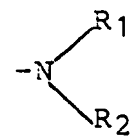

,

dans laquelle $R_1$ et $R_2$ peuvent être identiques ou différents, désignent un atome d'hydrogène, un radical alkyle inférieur en $C_1$-$C_8$, linéaire ou ramifié, substitué par au moins un groupement hydroxyle, ce radical alkyle inférieur en $C_1$-$C_8$ pouvant être interrompu par un ou plusieurs hétéroatomes choisis parmi l'oxygène, l'azote ou le soufre;

$R_1$ et $R_2$ ne désignant pas simultanément un atome d'hydrogène, $R_1$ et $R_2$ peuvent former avec l'atome d'azote auquel ils sont rattachés un hétérocycle comportant un ou plusieurs atomes d'azote, d'oxygène ou de soufre, l'hétérocycle étant éventuellement substitué par un groupement alkyle ou hydroxyalkyle, le groupement amino pouvant également provenir d'un sucre et leurs sels minéraux ou organiques;

caractérisé par le fait que l'on transforme l'acide eicosatétraynoïque-5,8,11,14 en chlorure d'acide ou en anhydride d'acide correspondant sur lequel on fait réagir en présence d'une amine tertiaire, une amine de formule :

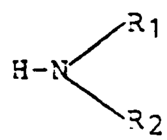

,

dans laquelle

$R_1$ et $R_2$ ont les significations indiquées ci-dessus, ou

que l'on fait réagir sur l'acide eicosatétraynoïque-5,8,11,14 le carbonyl diimidazole en présence d'un solvant et que l'on ajoute ensuite un excès d'amine de formule :

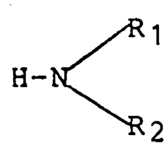

,

dans laquelle

$R_1$ et $R_2$ ont les significations indiquées dans la revendication 1.

**2.** Procédé selon la revendication 1, caractérisé par le fait que l'on prépare un composé répondant à la formule (I), dans lequel R est un groupement amino de structure :

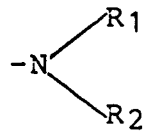

,

dans laquelle
$R_1$ désigne hydrogène et $R_2$ désigne un groupement :

$-CH_2-CH_2-OCH_2CH_2OH$, $-CH_2-CH_2OH$ $-CH_2-CHOH-CH_3$ ou $CH_2-CHOH-CH_2OH$

en mettant en oeuvre une amine de formule :

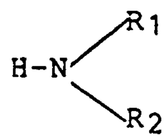

,

dans laquelle
$R_1$ et $R_2$ ont les significations indiquées ci-dessus.

**3.** Procédé selon la revendication 1, caractérisé par le fait que l'on prépare un composé répondant à la formule (I), dans laquelle R est un groupement amino de structure :

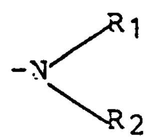

,

dans laquelle
$R_1$ et $R_2$ forment un cycle dérivé de la morpholine, de la pipérazine ou de l'(hydroxy-2' éthyl)-4 pipérazine en mettant en oeuvre une amine de formule :

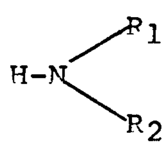

,

dans laquelle
$R_1$ et $R_2$ ont les significations indiquées ci-dessus.

**4.** Procédé de préparation selon la revendication 2, caractérisé par le fait que l'on prépare le N-(hydroxy-2 éthyloxyéthyl)eicosatétraynamide-5,8,11,14 répondant à la formule (I), dans laquelle R désigne le groupement hydroxy-2 éthyloxyéthylamino, en mettant en oeuvre l'hydroxy-2 éthyloxyéthylamine.

**5.** Procédé de préparation selon la revendication 2, caractérisé par le fait que l'on prépare le N-(hydroxy-2 éthyl)eicosatétraynamide-5,8,11,14 répondant à la formule (I) dans laquelle R désigne le groupement hydroxy-2 éthylamino en mettant en oeuvre l'éthanolamine.

**6.** Procédé de préparation selon la revendication 2, caractérisé par le fait que l'on prépare le N-(dihydroxy-2,3 propyl)eicosatétraynamide-5,8,11,14 répondant à la formule (I) dans laquelle R désigne le groupement dihydroxy-2,3 propylamino, en mettant en oeuvre l'amino-3 propanediol-1,2.

**7.** Procédé de préparation selon la revendication 3, caractérisé par le fait que l'on prépare l'(hydroxy-2'éthyl)-4 pipérazino-eicosatétraynamide-5,8,11,14 répondant à la formule (I) dans laquelle R désigne le

groupement (hydroxy-2'éthyl)-4 pipérazino en mettant en oeuvre l'hydroxy-2 éthyl pipérazine.

**8.** Procédé de préparation selon l'une des revendications précédentes, caractérisé en ce que l'acide eicosatétraynoïque-5,8,11,14 est préparé selon le procédé où on traite l'heptyne (1) répondant à la formule :

$C_5H_{11}\text{-}C{\equiv}C\text{-}H$ (1)

avec une base forte pour former l'acétylure correspondant qui est amené à réagir avec le dihalogéno-1,4 butyne-2 de formule :

$XCH_2\text{-}C{\equiv}C\text{-}CH_2X$ (2)

pour obtenir l'halogéno-1 undécadiyne-2,5 de formule :

$C_5H_{11}\text{-}C{\equiv}C\text{-}CH_2\text{-}C{\equiv}C\text{-}CH_2X$ (3)

que l'on amène à réagir avec le dianion de l'alcool propargylique, le produit résultant étant bromé par l'intermédiaire du tribromure de phosphore pour obtenir le bromo-1 tétradécatriyne-2,5,8 de formule :

$C_5H_{11}\text{-}C{\equiv}C\text{-}CH_2\text{-}C{\equiv}C\text{-}CH_2\text{-}C{\equiv}C\text{-}CH_2\text{-}Br$ (6)

qui est mis à réagir avec le dianion de l'acide hexyne-5 carboxylique pour obtenir l'acide eicosatétraynoïque.

**9.** Procédé de préparation d'une composition pharmaceutique destinée au traitement et à la prophylaxie des maladies allergiques et au traitement des dermatoses et des maladies inflammatoires telles que le psoriasis, l'eczema, l'acné, ou d'une composition cosmétique, caractérisé par le fait que l'on introduit dans un support ou diluant pharmaceutiquement ou cosmétiquement acceptable 0,01 à 10% en poids par rapport au poids total de la composition, et de préférence entre 0,1 et 5% en poids du composé répondant à la formule générale (I) :

$$C_5H_{11}\text{-}(C{\equiv}C\text{-}CH_2)_4\text{-}CH_2CH_2CO\ R \qquad (I)$$

dans laquelle :

R est un groupement amino de structure

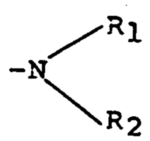 ,

dans laquelle

$R_1$ et $R_2$ peuvent être identiques ou différents, désignent un atome d'hydrogène, un radical alkyle inférieur en $C_1$-$C_8$, linéaire ou ramifié, substitué par au moins un groupement hydroxyle, ce radical alkyle inférieur en $C_1$-$C_8$ pouvant être interrompu par un ou plusieurs hétéroatomes choisis parmi l'oxygène, l'azote ou le soufre;

$R_1$ et $R_2$ ne désignant pas simultanément un atome d'hydrogène, $R_1$ et $R_2$ peuvent former avec l'atome d'azote auquel ils sont rattachés un hétérocycle comportant un ou plusieurs atomes d'azote, d'oxygène ou de soufre, l'hétérocycle étant éventuellement substitué par un groupement alkyle ou hydroxyalkyle, le groupement amino pouvant également provenir d'un sucre et leurs sels minéraux ou organiques.

**10.** Procédé selon la revendication 9, caractérisé par le fait que le groupement R dans la formule (I) désigne le groupement :

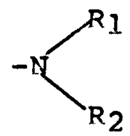 ,

dans laquelle

$R_1$ désigne hydrogène et $R_2$ un groupement :

- $CH_2$-$CH_2$-$OCH_2CH_2OH$, -$CH_2$-$CH_2OH$, - $CH_2$-CHOH-$CH_3$ ou $CH_2$-CHOH-$CH_2OH$

**11.** Procédé de préparation selon la revendication 9, caractérisé par le fait que $R_1$ et $R_2$ forment un cycle dérivé de la morpholine, de la pipérazine ou de l'hydroxy-2'-éthyl-4 pipérazine.

**12.** Procédé de préparation selon la revendication 10, caractérisé par le fait que le composé est le N-(hydroxy-2 éthyloxyéthyl)eicosatétraynamide-5,8,11,14.

**13.** Procédé de préparation selon la revendication 10, caractérisé par le fait que le composé est le N-(hydroxy-2 éthyl)eicosatétraynamide-5,8,11,14.

**14.** Procédé de préparation selon la revendication 10, caractérisé par le fait que le composé est le N-(dihydroxy-2,3 propyl)eicosatétraynamide-5,8,11,14.

**15.** Procédé selon la revendication 11, caractérisé par le fait que le composé est l'(hydroxy-2 éthyl)-4-pipérazino eicosatétraynamide-5,8,11,14.

**Claims**

**Claims for the following Contracting States : AT, BE, CH, DE, FR, GB, IT, LI, LU, NL, SE**

**1.** Compound characterised in that it is of the formula:

$$C_5H_{11}(C\equiv C-CH_2)_4CH_2CH_2CO\ R \qquad (I)$$

in which R is an amino group of structure

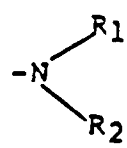

in which $R_1$ and $R_2$, which are identical or different, denote a hydrogen atom, a linear or branched lower $C_1$-$C_8$ alkyl radical which is substituted by at least one hydroxyl group, it being possible for this lower $C_1$-$C_8$ alkyl radical to be interrupted by one or more heteroatoms chosen from oxygen, nitrogen or sulphur, $R_1$ and $R_2$ not simultaneously denoting a hydrogen atom and it being possible for $R_1$ and $R_2$ to form with the nitrogen atom to which they are attached a heterocycle containing, as additional heteroatom, one or more nitrogen, oxygen or sulphur atoms, the heterocycle being optionally substituted by an alkyl or hydroxyalkyl group, it being also possible for the amino group to be derived from a sugar, and their salts with inorganic or organic acids.

**2.** Compound according to Claim 1, characterised in that it is of formula (I), in which R denotes the group:

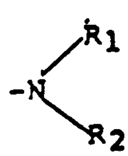

in which $R_1$ denotes hydrogen and $R_2$ a group:

$-CH_2CH_2-OCH_2CH_2OH, -CH_2CH_2OH, -CH_2-CHOHCH_3$ or $-CH_2CHOHCH_2OH$.

**3.** Compound according to Claim 1, characterised in that $R_1$ and $R_2$ form a ring derived from morpholine, piperazine and 4-(2'-hydroxyethyl)piperazine.

**4.** Compound according to Claim 2, characterised in that it is N-(2-hydroxyethyloxyethyl)-5,8,11,14-eicosatetraynamide.

**5.** Compound according to Claim 2, characterised in that it is N-(2-hydroxyethyl)-5,8,11,14-eicosatetraynamide.

**6.** Compound according to Claim 2, characterised in that it is N-(2,3-dihydroxypropyl)-5,8,11,14-eicosatetraynamide.

**7.** Compound according to Claim 3, characterised in that it is 4'(2-hydroxyethyl)piperazino-5,8,11,14-eicosatetraynamide.

**8.** Process for preparing the compounds according to any one of Claims 1 to 7, characterised in that 5,8,11,14-eicosatetraynoic acid is converted to the corresponding acid chloride or acid anhydride which is reacted, in the presence of a tertiary amine, with an amine of formula:

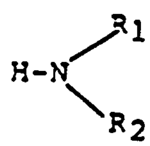

in which $R_1$ and $R_2$ have the meanings indicated in Claim 1, or
in that 5,8,11,14-eicosatetraynoic acid is reacted with carbonyldiimidazole in the presence of a solvent, and in that excess amine of formula:

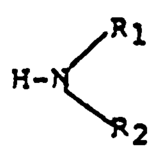

in which $R_1$ and $R_2$ have the meanings indicated in Claim 1, is then added.

**9.** Preparation process according to Claim 8, characterised in that the 5,8,11,14-eicosatetraynoic acid used is prepared according to a process where 1-heptyne of formula:

$C_5H_{11}-C{\equiv}C-H$ (1)

is treated with a strong base in order to form the corresponding acetylide which is reacted with the 1,4-dihalogeno-2-butyne of formula:

$XCH_2-C{\equiv}C-CH_2X$ (2)

in order to obtain the 1-halogeno-2,5-undecadiyne of formula:

$C_5H_{11}-C{\equiv}C-CH_2-C{\equiv}C-CH_2X$ (3)

which is reacted with the dianion of propargyl alcohol, the resulting product being brominated by means of phosphorus tribromide in order to obtain the 1-bromo-2,5,8-tetradecatriyne of formula:

$C_5H_{11}-C{\equiv}C-CH_2-C{\equiv}C-CH_2-C{\equiv}C-CH_2-Br$ (6)

which is reacted with the dianion of 5-hexynecarboxylic acid in order to obtain eicosatetraynoic acid.

**10.** Medicinal product characterised in that it contains a compound as defined in any one of Claims 1 to 7.

**11.** Pharmaceutical composition intended to be administered systemically or locally, characterised in that it contains, in the presence of a pharmaceutically acceptable carrier or diluent, at least one compound as defined in any one of Claims 1 to 7.

**12.** Pharmaceutical composition intended to be administered topically, which is provided in the form of creams, tinctures, ointments, pommades, powders, patches, impregnated pads, solutions, gels, lotions, sprays or suspensions, characterised in that it contains at least one compound as defined in any one of Claims 1 to 7.

**13.** Pharmaceutical composition which is provided in the form of a composition intended to be administered in a parenteral manner, intravenously, interperitoneally, intramuscularly, subcutaneously or intradermally, characterised in that it contains a compound as defined in any one of Claims 1 to 7, in a pharmaceutically acceptable diluent.

**14.** Pharmaceutical composition intended to be administered enterally, which is provided in the form of tablets, hard gelatine capsules, lozenges, syrups, suspensions, solutions, powders, granules or emulsions, characterised in that it contains a compound as defined in any one of Claims 1 to 7.

**15.** Pharmaceutical composition according to any one of Claims 11 to 13, characterised in that it contains the active compound of formula (I) in proportions of 0.01 to 10% by weight relative to the total weight of the composition, and preferably between 0.1 and 5% by weight.

**16.** Use of a compound as defined in any one of Claims 1 to 7, for the preparation of a medicinal product intended for the treatment and prevention of allergic diseases and for the treatment of dermatoses and inflammatory diseases such as psoriasis, eczema and acne.

**17.** Use according to Claim 16, of a compound defined in any one of Claims 1 to 7, for the preparation of a medicinal product intended for the treatment and prevention of allergic diseases and for the treatment of dermatoses and inflammatory diseases such as psoriasis, eczema and acne, at doses of 0.05 to 500 mg/kg/day, and preferably 0.5 to 100 mg/kg/day.

**18.** Cosmetic composition characterised in that it contains at least one compound as defined in any one of Claims 1 to 7, in a proportion of between 0.01 and 10% by weight, and preferably between 0.1 and 5% by weight, relative to the total weight of the composition, in the presence of a cosmetically acceptable carrier.

**19.** Application of a compound as defined in any one of Claims 1 to 7, as a cosmetic product.

**Claims for the following Contracting States : ES, GR**

**1.** Process for preparing the compounds of general formula (I):

$$C_5H_{11}(C{\equiv}C-CH_2)_4-CH_2CH_2CO\ R \qquad (I)$$

in which:

R is an amino group of structure

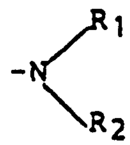

in which $R_1$ and $R_2$ may be identical or different and denote a hydrogen atom, a linear or branched lower $C_1$-$C_8$ alkyl radical which is substituted by at least one hydroxyl group, it being possible for this lower $C_1$-$C_8$ alkyl radical to be interrupted by one or more heteroatoms chosen from oxygen, nitrogen or sulphur;

$R_1$ and $R_2$ not simultaneously denoting a hydrogen atom, $R_1$ and $R_2$ can form with the nitrogen atom to which they are attached a heterocycle containing one or more nitrogen, oxygen or sulphur atoms, the heterocycle being optionally substituted by an alkyl or hydroxyalkyl group, it being also possible for the amino group to be derived from a sugar, and their inorganic or organic salts;

characterised in that 5,8,11,14-eicosatetraynoic acid is converted to the corresponding acid chloride or acid anhydride which is reacted, in the presence of a tertiary amine, with an amine of formula:

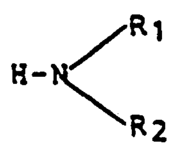

in which $R_1$ and $R_2$ have the meanings indicated above, or

in that 5,8,11,14-eicosatetraynoic acid is reacted with carbonyl diimidazole in the presence of a solvent, and in that excess amine of formula:

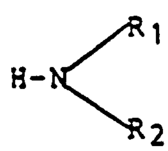

in which $R_1$ and $R_2$ have the meanings indicated in Claim 1, is then added.

**2.** Process according to Claim 1, characterised in that a compound of formula (I), in which R is an amino group of structure:

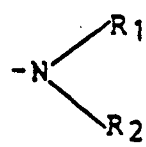

in which $R_1$ denotes hydrogen and $R_2$ denotes a group:

$-CH_2-CH_2-OCH_2CH_2OH$, $-CH_2-CH_2OH$ $-CH_2-CHOH-CH_3$ or $CH_2-CHOH-CH_2OH$,

is prepared using an amine of formula:

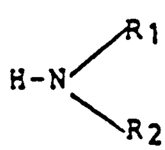

in which $R_1$ and $R_2$ have the meanings indicated above.

**3.** Process according to Claim 1, characterised in that a compound of formula (I), in which R is an amino group of structure:

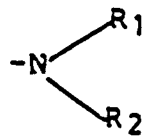

in which $R_1$ and $R_2$ form with a ring derived from morpholine, piperazine or 4-(2'-hydroxyethyl)-piperazine, is prepared using an amine of formula:

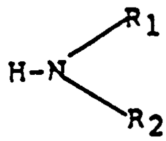

in which $R_1$ and $R_2$ have the meanings indicated above.

**4.** Preparation process according to Claim 2, characterised in that N-(2-hydroxyethyloxyethyl)-5,8,11,14-eicosatetraynamide of formula (I), in which R denotes a 2-hydroxyethyloxyethylamino group, is prepared using 2-hydroxyethyloxyethylamine.

**5.** Preparation process according to Claim 2, characterised in that N-(2-hydroxyethyl)-5,8,11,14-eicosatetraynamide of formula (I), in which R denotes a 2-hydroxyethylamino group, is prepared using ethanolamine.

**6.** Preparation process according to Claim 2, characterised in that N-(2,3-dihydroxypropyl)-5,8,11,14-eicosatetraynamide of formula (I), in which R denotes a 2,3-dihydroxypropylamino group, is prepared using 3-amino-1,2-propanediol.

**7.** Preparation process according to Claim 3, characterised in that 4-(2'-hydroxyethyl)piperazino-5,8,11,14-eicosatetraynamide of formula (I), in which R denotes a 4-(2'-hydroxyethyl)piperazino group, is prepared using 2-hydroxyethylpiperazine.

**8.** Preparation process according to one of the preceding claims, characterised in that 5,8,11,14-eicosatetraynoic acid is prepared according to a process where the heptyne (1) of formula:

$C_5H_{11}$-C≡C-H (1)

is treated with a strong base in order to form the corresponding acetylide which is reacted with the 1,4-dihalogeno-2-butyne of formula:

$XCH_2$-C≡C-$CH_2X$ (2)

in order to obtain the 1-halogeno-2,5-undecadiyne of formula:

$C_5H_{11}$-C≡C-$CH_2$-C≡C-$CH_2X$ (3)

which is reacted with the dianion of propargyl alcohol, the resulting product being brominated by means of phosphorus tribromide in order to obtain the 1-bromo-2,5,8-tetradecatriyne of formula:

$C_5H_{11}$-C≡C-$CH_2$-C≡C-$CH_2$-C≡C-$CH_2$-Br (6)

which is reacted with the dianion of 5-hexynecarboxylic acid in order to obtain eicosatetraynoic acid.

**9.** Process for preparing a pharmaceutical composition intended for the treatment and prevention of allergic diseases and for the treatment of dermatoses and inflammatory diseases such as psoriasis, eczema and acne, or a cosmetic composition, characterised in that there is introduced into a pharmaceutically or cosmetically acceptable carrier or diluent, 0.01 to 10% by weight relative to the total weight of the composition, and preferably between 0.1 and 5% by weight, of the compound of general formula (I):

$$C_5H_{11}-(C\equiv C-CH_2)_4-CH_2CH_2CO\ R \qquad (I)$$

in which:

R is an amino group of structure

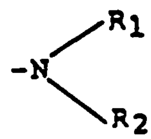

in which $R_1$ and $R_2$ may be identical or different and denote a hydrogen atom, a linear or branched lower $C_1$-$C_8$ alkyl radical which is substituted by at least one hydroxyl group, it being possible for this lower $C_1$-$C_8$ alkyl radical to be interrupted by one or more heteroatoms chosen from oxygen, nitrogen or sulphur;

$R_1$ and $R_2$ not simultaneously denoting a hydrogen atom, $R_1$ and $R_2$ can form with the nitrogen atom to which they are attached a heterocycle containing one or more nitrogen, oxygen or sulphur atoms, the heterocycle being optionally substituted by an alkyl or hydroxyalkyl group, it being also possible for the amino group to be derived from a sugar, and their inorganic or organic salts.

**10.** Process according to Claim 9, characterised in that the R group in the formula (I) denotes the group:

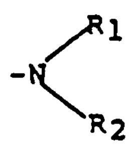

in which $R_1$ denotes hydrogen and $R_2$ a group:

$-CH_2-CH_2-OCH_2CH_2OH$, $-CH_2-CH_2OH$, $-CH_2-CHOH-CH_3$ or $CH_2-CHOH-CH_2OH$.

**11.** Preparation process according to Claim 9, characterised in that $R_1$ and $R_2$ form a ring derived from morpholine, piperazine or 4-(2'-hydroxyethyl)piperazine.

**12.** Preparation process according to Claim 10, characterised in that the compound is N-(2-hydroxyethyloxyethyl)-5,8,11,14-eicosatetraynamide.

**13.** Preparation process according to Claim 10, characterised in that the compound is N-(2-hydroxyethyl)-5,8,11,14-eicosatetraynamide.

**14.** Preparation process according to Claim 10, characterised in that the compound is N-(2,3-dihydroxypropyl)-5,8,11,14-eicosatetraynamide.

**15.** Process according to Claim 11, characterised in that the compound is 4-(2-hydroxyethyl)piperazino-5,8,11,14-eicosatetraynamide.

**Patentansprüche**

**Patentansprüche für folgende Vertragsstaaten : AT, BE, CH, DE, FR, GB, IT, LI, LU, NL, SE**

1. Verbindung der Formel:

$$C_5H_{11}(C{\equiv}C\text{-}CH_2)_4CH_2CH_2CO\ R \qquad (I)$$

worin R eine Aminogruppe der Struktur

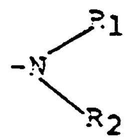

ist, worin $R_1$ und $R_2$, gleich oder verschieden, ein Wasserstoffatom, einen linearen oder verzweigten, durch mindestens eine Hydroxylgruppe substituierten $C_1$-$C_8$-Niedrigalkylrest, der durch eines oder mehrere unter Sauerstoff, Stickstoff oder Schwefel ausgewählte Heteroatome unterbrochen sein kann, bedeuten, wobei $R_1$ und $R_2$ nicht gleichzeitig ein Wasserstoffatom darstellen, und wobei $R_1$ und $R_2$ mit dem Stickstoffatom, an das sie gebunden sind, einen Heterozyklus bilden können, der als zusätzliches Heteroatom eines oder mehrere Atome von Stickstoff, Sauerstoff oder Schwefel aufweisen kann und ggf. durch eine Alkyl- oder Hydroxyalkylgruppe substituiert ist, wobei die Aminogruppe auch aus einem Zuckerrest abgeleitet sein kann,
sowie deren Salze von Mineralsäuren oder organischen Säuren.

2. Verbindung gemäß Anspruch 1,
dadurch **gekennzeichnet,** daß
sie der Formel (I) entspricht, worin R die Gruppe bedeutet,
worin $R_1$ Wasserstoff und $R_2$ eine Gruppe darstellen:

$-CH_2CH_2-OCH_2CH_2OH, -CH_2CH_2OH, -CH_2-CHOHCH_3$ oder $-CH_2CHOHCH_2OH$.

3. Verbindung gemäß Anspruch 1,
dadurch **gekennzeichnet,** daß
$R_1$ und $R_2$ einen Ring bilden, der von Morpholin, Piperazin und 4-(2'-Hydroxyethyl)piperazin abgeleitet ist.

4. Verbindung gemäß Anspruch 2,
dadurch **gekennzeichnet,** daß
sie N-(2-Hydroxyethyloxyethyl)eicosatetrain-5,8,11,14-säureamid ist.

5. Verbindung gemäß Anspruch 2,
dadurch **gekennzeichnet,** daß
sie N-(2-Hydroxyethyl)eicosatetrain-5,8,11,14-säureamid ist.

6. Verbindung gemäß Anspruch 2,
dadurch **gekennzeichnet,** daß
sie N-(2,3-Dihydroxypropyl)eicosatetrain-5,8,11,14-säureamid ist.

7. Verbindung gemäß Anspruch 3,
dadurch **gekennzeichnet,** daß
sie 4'-(2-Hydroxyethyl)piperazinoeicosatetrain-5,8,11,14-säureamid ist.

8. Verfahren zur Herstellung von Verbindungen gemäß jedem der Ansprüche 1 bis 7,
dadurch **gekennzeichnet,** daß
man Eicosatetrain-5,8,11,14-säure in das entsprechende Säurechlorid oder -anhydrid überführt, mit

welchen man in der Gegenwart eines tertiären Amins ein Amin der Formel reagieren läßt:

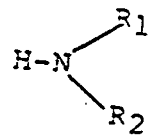

worin $R_1$ und $R_2$ die in Anspruch 1 angegebenen Bedeutungen haben, oder daß man Carbonyldiimidazol in der Gegenwart eines Lösungsmittels mit Eicosatetrain-5,8,11,14-säure reagieren läßt und danach einen Überschuß eines Amins der Formel zufügt:

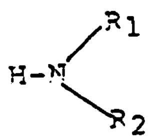

worin $R_1$ und $R_2$ die in Anspruch 1 angegebenen Bedeutungen haben.

**9.** Herstellungsverfahren gemäß Anspruch 8,
dadurch **gekennzeichnet,** daß
man Eicosatetrain-5,8,11,14-säure einsetzt, die durch ein Verfahren erhältlich ist, bei dem man Heptin-1 der Formel:

$C_5H_{11}$-C≡C-H (1)

mit einer starken Base behandelt, um das entsprechende Acetylid zu bilden, das man mit 1,4-Dihalogenbutin-2 der Formel reagieren läßt:

$XCH_2$-C≡C-$CH_2X$ (2)

um 1-Halogenundecadiin-2,5 der Formel zu erhalten:

$C_5H_{11}$-C≡C-$CH_2$-C≡C-$CH_2X$ (3)

das man mit dem Dianion des Propargylalkohols reagieren läßt, wobei das entstandene Produkt als Zwischenprodukt mit Phosphortribromid bromiert wird, um 1-Bromtetradecatriin-2,5,8 der Formel zu erhalten:

$C_5H_{11}$-C≡C-$CH_2$-C≡C-$CH_2$-C≡C-$CH_2$-Br (6)

das mit dem Dianion der Hexin-5-carboxylsäure zur Reaktion gebracht wird, um die Eicosatetrainsäure zu erhalten.

**10.** Medikament, dadurch **gekennzeichnet,** daß
es eine Verbindung gemäß jedem der Ansprüche 1 bis 7 enthält.

**11.** Pharmazeutische Zusammensetzung zur Verabreichung auf systemischem oder lokalem Weg,
dadurch **gekennzeichnet,** daß
sie in der Gegenwart eines pharmazeutisch verträglichen Trägers oder Verdünnungsmittels mindestens eine Verbindung gemäß jedem der Ansprüche 1 bis 7 enthält.

**12.** Pharmazeutische Zusammensetzung zur Verabreichung auf topischem Weg, welche in Form von Cremes, Tinkturen, Salben, Pommaden, Pudern, Tupfern, getränkten Tampons, Lösungen, Gelen, Lotionen, Sprays, Suspensionen vorliegt,
dadurch **gekennzeichnet,** daß
sie mindestens eine Verbindung gemäß jedem der Ansprüche 1 bis 7 enthält.

**13.** Pharmazeutische Zusammensetzung, die in Form einer Zusammensetzung vorliegt, die dazu bestimmt ist, auf intravenösem, intraperitonealem, intramuskulärem, subkutanem oder intradermischem Weg verabreicht zu werden,
dadurch **gekennzeichnet,** daß
sie eine Verbindung gemäß jedem der Ansprüche 1 bis 7 in einem pharmazeutisch verträglichen Verdünnungsmittel enthält.

**14.** Pharmazeutische Zusammensetzung zur Verabreichung auf enteralem Weg, welche in Form von Tabletten, Kapseln, Dragees, Sirups, Suspensionen, Lösungen, Pulvern, Körnchen, Emulsionen vorliegt,
dadurch **gekennzeichnet,** daß
sie eine Verbindung gemäß jedem der Ansprüche 1 bis 7 enthält.

**15.** Pharmazeutische Zusammensetzung gemäß jedem der Ansprüche 11 bis 13,
dadurch **gekennzeichnet,** daß
sie die Wirkverbindung der Formel (I) in Mengen von 0,01 bis 10 Gewichtsprozent, vorzugsweise 0,1 bis 5 Gew.%, bezogen auf Gesamtgewicht der Zusammensetzung, enthält.

**16.** Verwendung einer Verbindung gemäß jedem der Ansprüche 1 bis 7 zur Herstellung eines Medikaments zur Behandlung und Prophylaxe von Allergiekrankheiten und zur Behandlung von Dermatosen und Entzündungen wie Psoriasis, Ekzemen und Akne.

**17.** Verwendung gemäß Anspruch 16 einer Verbindung gemäß jedem der Ansprüche 1 bis 7 zur Herstellung eines Medikaments zur Behandlung und Prophylaxe von Allergiekrankheiten und zur Behandlung von Dermatosen und Entzündungen wie Psoriases, Ekzemen und Akne in Dosierungsmengen von 0,05 bis 500 mg/kg/Tag, vorzugsweise 0,5 bis 100 mg/kg/Tag.

**18.** Kosmetische Zusammensetzung,
dadurch **gekennzeichnet,** daß
sie in Gegenwart eines kosmetisch verträglichen Trägers mindestens eine Verbindung gemäß jedem der Ansprüche 1 bis 7 in Mengen von 0,01 bis 10 Gew.%, vorzugsweise 0,1 bis 5 Gew.%, bezogen auf Gesamtgewicht der Zusammensetzung, enthält.

**19.** Anwendung einer Verbindung gemäß jedem der Ansprüche 1 bis 7 als Kosmetikprodukt.

**Patentansprüche für folgende Vertragsstaaten : ES, GR**

**1.** Verfahren zur Herstellung von Verbindungen der allgemeinen Formel:

$$C_5H_{11}(C\equiv C-CH_2)_4CH_2CH_2CO\ R \qquad (I)$$

worin R eine Aminogruppe der Struktur

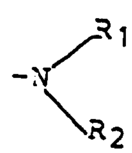

ist, worin $R_1$ und $R_2$, gleich oder verschieden, ein Wasserstoffatom, einen linearen oder verzweigten, durch mindestens eine Hydroxylgruppe substituierten $C_1$-$C_8$-Niedrigalkylrest, der durch eines oder mehrere unter Sauerstoff, Stickstoff oder Schwefel ausgewählte Heteroatome unterbrochen sein kann, bedeuten, wobei $R_1$ und $R_2$ nicht gleichzeitig ein Wasserstoffatom darstellen, und wobei $R_1$ und $R_2$ mit dem Stickstoffatom, an das sie gebunden sind, einen Heterozyklus bilden können, der als zusätzliches Heteroatom eines oder mehrere Atome von Stickstoff, Sauerstoff oder Schwefel aufweisen kann und ggf. durch eine Alkyl- oder Hydroxyalkylgruppe substituiert ist, wobei die Aminogruppe auch aus einem

Zuckerrest abgeleitet sein kann,
sowie von Salzen von Mineralsäuren oder organischen Säuren davon,
dadurch **gekennzeichnet,** daß
man Eicosatetrain-5,8,11,14-säure in das entsprechende Säurechlorid oder -anhydrid überführt, mit welchen man in der Gegenwart eines tertiären Amins ein Amin der Formel reagieren läßt:

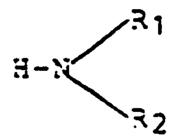

worin $R_1$ und $R_2$ die in Anspruch 1 angegebenen Bedeutungen haben, oder
daß man Carbonyldiimidazol in der Gegenwart eines Lösungsmittels mit Eicosatetrain-5,8,11,14-säure reagieren läßt und danach einen Überschuß eines Amins der Formel zufügt:

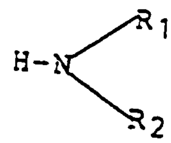

worin $R_1$ und $R_2$ die oben angegebenen Bedeutungen haben.

**2.** Verfahren gemäß Anspruch 1,
dadurch **gekennzeichnet,** daß
man eine Verbindung der Formel (I) herstellt, worin R eine Aminogruppe der Struktur ist:

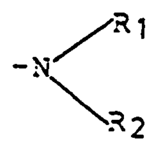

worin
$R_1$ Wasserstoff und $R_2$ eine Gruppe darstellt:

$-CH_2-CH_2-OCH_2CH_2OH$, $-CH_2-CH_2OH$ $-CH_2-CHOH-CH_3$ oder $CH_2-CHOH-CH_2OH$

wobei man ein Amin der Formel einsetzt:

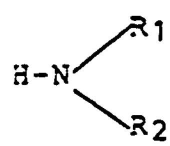

worin
$R_1$ und $R_2$ die oben angegebenen Bedeutungen haben.

**3.** Verfahren gemäß Anspruch 1,
dadurch **gekennzeichnet,** daß
man eine Verbindung der Formel (I) herstellt, worin R eine Aminogruppe der Struktur ist:

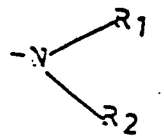

worin
$R_1$ und $R_2$ einen Ring bilden, der von Morpholin, Piperazin oder 4-(2'-Hydroxyethyl)piperazin abgeleitet ist, wobei man ein Amin der Formel einsetzt:

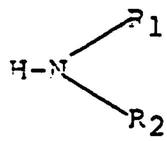

worin
$R_1$ und $R_2$ die oben angegebenen Bedeutungen haben.

**4.** Herstellungsverfahren gemäß Anspruch 2,
dadurch **gekennzeichnet,** daß
man N-(2-Hydroxyethyloxyethyl)eicosatetrain-5,8,11,14-säureamid der Formel (I), worin R die 2-Hydroxyethyloxyethylaminogruppe bedeutet, herstellt, indem man 2-Hydroxyethyloxyethylamin einsetzt.

**5.** Herstellungsverfahren gemäß Anspruch 2,
dadurch **gekennzeichnet,** daß
man N-(2-Hydroxyethyl)eicosatetrain-5,8,11,14-säureamid der Formel (I), worin R die 2-Hydroxyethylaminogruppe bedeutet, herstellt, indem man Ethanolamin einsetzt.

**6.** Herstellungsverfahren gemäß Anspruch 2,
dadurch **gekennzeichnet,** daß
man N-(2,3-Dihydroxypropyl)eicosatetrain-5,8,11,14-säureamid der Formel (I), worin R die 2,3-Dihydroxypropylaminogruppe bedeutet, herstellt, indem man 3-Aminopropandiol-1,2 einsetzt.

**7.** Herstellungsverfahren gemäß Anspruch 3,
dadurch **gekennzeichnet,** daß
man 4-(2'-Hydroxyethyl)piperazinoeicosatetrain-5,8,11,14-säureamid der Formel (I), worin R die 4'-(2-Hydroxyethyl)piperazinogruppe bedeutet, herstellt, indem man 2-Hydroxyethylpiperazin einsetzt.

**8.** Verfahren gemäß jedem der vorhergehenden Ansprüche,
dadurch **gekennzeichnet,** daß
Eicosatetrain-5,8,11,14-säure gemäß eines Verfahrens hergestellt wird, wobei man Eicosatetrain-5,8,11,14-säure einsetzt, die durch ein Verfahren erhältlich ist, bei dem man Heptin-1 der Formel:

$C_5H_{11}$-C≡C-H (1)

mit einer starken Base behandelt, um das entsprechende Acetylid zu bilden, das man mit 1,4-Dihalogenbutin-2 der Formel reagieren läßt:

$XCH_2$-C≡C-$CH_2X$ (2)

um 1-Halogenundecadiin-2,5 der Formel zu erhalten:

$C_5H_{11}$-C≡C-$CH_2$-C≡C-$CH_2X$ (3)

das man mit dem Dianion des Propargylalkohols reagieren läßt, wobei das entstandene Produkt als Zwischenprodukt mit Phosphortribromid bromiert wird, um 1-Bromtetradecatriin-2,5,8 der Formel zu

erhalten:

$C_5H_{11}-C\equiv C-CH_2-C\equiv C-CH_2-C\equiv C-CH_2-Br$ (6)

das mit dem Dianion der Hexin-5-carboxylsäure zur Reaktion gebracht wird, um die Eicosatetrainsäure zu erhalten.

**9.** Verfahren zur Herstellung einer pharmazeutischen Zusammensetzung zur Behandlung und Prophylaxe von Allergiekrankheiten und zur Behandlung von Dermatosen und Entzündungen wie Psoriases, Ekzemen und Akne oder zur Herstellung einer kosmetischen Zusammensetzung,
dadurch **gekennzeichnet,** daß
man in einen pharmazeutisch oder kosmetisch verträglichen Träger oder ein entsprechendes Verdünnungsmittel 0,01 bis 10 Gew.%, vorzugsweise 0,1 bis 5 Gew.%, bezogen auf Gesamtgewicht der Zusammensetzung, einer Verbindung der allgemeinen Formel (I) einbringt:

$$C_5H_{11}-(C\equiv C-CH_2)_4-CH_2CH_2CO\ R \qquad (I)$$

worin R eine Aminogruppe der Struktur

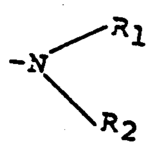

ist, worin $R_1$ und $R_2$, gleich oder verschieden, ein Wasserstoffatom, einen linearen oder verzweigten, durch mindestens eine Hydroxylgruppe substituierten $C_1$-$C_8$-Niedrigalkylrest, der durch eines oder mehrere unter Sauerstoff, Stickstoff oder Schwefel ausgewählte Heteroatome unterbrochen sein kann, bedeuten, wobei $R_1$ und $R_2$ nicht gleichzeitig ein Wasserstoffatom darstellen, und wobei $R_1$ und $R_2$ mit dem Stickstoffatom, an das sie gebunden sind, einen Heterozyklus bilden können, der als zusätzliches Heteroatom eines oder mehrere Atome von Stickstoff, Sauerstoff oder Schwefel aufweisen kann und ggf. durch eine Alkyl- oder Hydroxyalkylgruppe substituiert ist, wobei die Aminogruppe auch aus einem Zuckerrest abgeleitet sein kann,
sowie von Salzen von Mineralsäuren oder organischen Säuren davon.

**10.** Verfahren gemäß Anspruch 9,
dadurch **gekennzeichnet,** daß
die Gruppe R in der Formel (I) die Gruppe bedeutet:

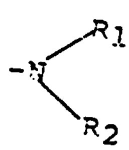

worin
$R_1$ Wasserstoff und $R_2$ eine Gruppe darstellen:

$- CH_2-CH_2-OCH_2CH_2OH$, $-CH_2-CH_2OH$, $- CH_2-CHOH-CH_3$ $CH_2-CHOH-CH_2OH$

**11.** Herstellungsverfahren gemäß Anspruch 9,
dadurch **gekennzeichnet,** daß
$R_1$ und $R_2$ einen Ring bilden, der von Morpholin, Piperazin oder 4-(2'-Hydroxyethyl)piperazin abgeleitet ist.

**12.** Herstellungsverfahren gemäß Anspruch 10,
dadurch **gekennzeichnet,** daß
die Verbindung N-(2-Hydroxyethyloxyethyl)eicosatetrain-5,8,11,14-säureamid ist.

**13.** Herstellungsverfahren gemäß Anspruch 10,
dadurch **gekennzeichnet,** daß
die Verbindung N-(2-Hydroxyethyl)eicosatetrain-5,8,11,14-säureamid ist.

**14.** Herstellungsverfahren gemäß Anspruch 10,
dadurch **gekennzeichnet,** daß
die Verbindung N-(2,3-Dihydroxypropyl)eicosatetrain-5,8,11,14-säureamid ist.

**15.** Verfahren gemäß Anspruch 11,
dadurch **gekennzeichnet,** daß
die Verbindung 4'-(2-Hydroxyethyl)piperazinoeicosatetrain-5,8,11,14-säureamid ist.